# EUROPEAN PATENT APPLICATION

(11) **EP 2 368 974 A1**
(43) Date of publication of application: **28.09.2011**
(21) Application number: 09818766.9
(22) Date of filing: 18.09.2009
(51) Int. Cl.: C12N 5/0775, C12N 5/073, A61K 8/64, A61K 8/98, A61Q 19/00, A61K 35/48, A61P 17/00

(54) **METHODS FOR ISOLATING MESENCHYMAL STEM CELLS FROM EMBRYOS OF HUMAN OR ANIMALS AND EXTRACTING SECRETION SUBSTANCES THEREOF**

(30) Priority: 10.10.2008 WO PCT/CN2008/072648
(71) Applicant: Team Youn Biomedical Technology Co., Ltd., Shenzhen, Guangdong 518000 (CN)
(72) Inventor: LIAN, Qizhou, Shenzhen Guangdong 518000 (CN); XIA, Jianchuan, Shenzhen Guangdong 518000 (CN)
(74) Representative: Hämmalov, Juhan
(86) International application number: PCT/CN2009/074031
(87) International publication number: WO 2010/040302

(57) **Abstract**

The present invention relates to a method of extracting mesenchymal stem cells from human or animal embryo, comprising: A. taking the sample based on human or animal embryo, centrifuging the sample; B. digesting the sample with enzyme digestion solution, and making single cell suspension, wherein said enzyme digestion solution comprises trysin-EDTA and collagenase IV; C. adding mesenchymal stem cell culture medium A to the isolated single cells, inoculating and culturing the cells; D. treating the cells with adherence purification, and obtaining mesenchymal stem cells by magnetic sorting. The present invention also relates to a method of extracting the secretion product from mesenchymal stem cells, the use of the secretion product in the aspects of cosmetic and healthcare, and an epidermal cell repairing lotion made using the secretion product. In the process according to the present invention of extracting mesenchymal stem cells from human or animal embryo, the mixture of trypsin-EDTA and collagenase IV is used as the enzyme digestion solution, enabling to obtain the maximal number of single cells, while reducing cell damage to the lowest level.

## Description

### Technical Field

The present invention relates to the field of cell biology. More specifically, It relates to a method for extracting mesenchymal stem cell from human or animal embryo and for extracting the secretion product thereof.

### Background Art

Mesenchymal stem cells (MSC) are a kind of tissue stem cells that are multipotent and have self-renewal ability. They can differentiate into various cell types: osteocybe, chondrocyte, fat cell, and neuronal cell etc.. They were initially isolated from bone marrow. In recent years mesenchymal cells have been isolated from tissues such as bone marrow, peripheral blood, compact bone, cartilage and muscle. The common feature of these tissues is that they are formed by mesenchyme and blood vessels originated from extraembryonic mesoderm. Mesenchymal stem cells are easily isolated and purified from bone marrow for effective *in vitro* amplification, and they have the functions of improving bone marrow environment and facilitating hematopoitic reconstitution. In the aspect of immune property, mesenchymal stem cells do not express or only express at negligible level molecules of the MHCII type. Mesenchymal stem cells from unrelated donor do not cause allogenic lymphocyte reaction and can downregulate allogenic immune response. Because of having the properties described above, upon its discovery, MSC rapidly becomes the Ideal cell source in the aspects of cell therapy, gene therapy *etc*. and important seed cell In tissue engineering for the repair of bone or cartilage injury.

Currently reported mesenchymal stem cells mainly come from bone marrow and are obtained using density gradient separation. The donor has to go through a rather painful operation for the marrow to be taken and during the material-taken process and afterwards, there would be a very high chance of infection. Additionally, since the content of mesenchymal stem cells in human bone marrow Is extremely scarce, about only one mesenchymal stem cell In every 10⁵-10⁶ mononuclear cells, and importantly the increase of age or In combination with diseases such as infection and tumor, the number of mesenchymal stem cells within bone marrow and their proliferation and differentiation ability decrease significantly, which makes them greatly limited in research and application, in particular clinical application.

Conventional method of isolating mesenchymal stem cells comprises the following steps: (1) blood is Immediately released after the placenta is expulsed from the uterus and sample is obtained; (2) single cell suspension is obtained by continuous infusion or collagenase digestion; (3) the single cell suspension Is subjected to density gradient centrifugation or immunomagnetic separation to obtain mesenchymal stem cells. The productivity of this method is relatively low, making it unable to obtain large amount of mesenchymal stem cells in a short time and the purity of the cells is relatively low; in addition, cells are quite seriously damaged by the treatment process, making it difficult to assure the quality of the mesenchymal stem cells; Furthermore, due to the low productivity, it is also difficult to extract secretion products with good prospect for application.

Therefore, to rapidly obtain large quantities of mesenchymal stem cells of high purity and high quality, a new method for extracting mesenchymal stem cells from human or animal embryos is desired.

### Disclosure of the Invention

One of the objects of the present invention is to provide a method for extracting mesenchymal stem cell from human or animal embryo and for extracting the secretion product thereof, intending to solve the problems existing in the prior art of low productivity and purity, and being difficult to assure cell quality.

To accomplish the objective of the invention, said method for extracting mesenchymal stem cells from human or animal embryo comprises the following steps: A. a human or animal embryo derived sample is obtained and treated with centrifugation; B. the sample is digested with enzyme digestion solution to make single cell suspension, wherein said enzyme digestion solution contains tripsin-EDTA and collagenase IV; C. Mesenchymal stem cell culture medium A is added Into the isolated single cells to inoculate and culture the cells; D. the cells are treated with adherence purification and mesenchymal stem cells are obtained by magnetic sorting.

Preferably, the enzyme digestion solution in said step B comprises: tripsin 0.125g/L- 0.375g/L; EDTA 0.075g/L - 0.125g/L and collagenase IV 0.25g/L - 0.75g/L.

The preparation of the enzyme digestion solution in said step B comprises: mixing 100ml D-HBSS containing 0.05g tripsin and 0.02g EDTA·4Na with 100ml normal saline containing 0.01g collagenase IV at the volume ratio of 1:1.

Preferably, said mesenchymal stem cell culture medium A comprises:

| | |
|---|---|
| Basic fibroblast growth factor | 1 ng/ml-100 ng/ml medium |
| Epidermal growth factor | 1 ng/ml - 100 ng/ml medium |
| Platelet derived growth factor | 1 ng/ml- 100 ng/ml medium |

wherein, said medium comprises components of the following weight percentage:

| | |
|---|---|
| DMEM medium | 68%-97% |
| Umbilical cord blood plasma | 1%-30% |
| Antibiotic and glutamine | 1% |
| Non-essential amino acid | 1% |
| Antioxidant | 0-0.1% |

Preferably, the adherence purification treatment in said step D comprises:
taking out non-adherent cells with a pipette, adding in normal saline to wash and changing to new mesenchymal stem cell culture medium A to continue culturing; digesting with tripain-EDTA when the adherent cells expand to reach the set volume of the culture flask to obtain single cell suspension.

Preferably, the magnetic sorting in said step D comprises:
using immunomagnetic separation method to select CD271 and CD73 double-positive cell population from said single cells, namely the mesenchymal stem cells isolated from embryos.

Preferably, It also comprises the following steps after said step D:
E. propagate and passage the obtained mesenchymal stem cells; and/or
F. extract the secretion product of the Mesenchymal stem cells.

Preferably, said step F comprises:
F1. Digest the mesenchymal stem cells with enzyme digestion solution
F2. Add culture medium into the collected cells for inoculation and culturing, and add in mesenchymal stem cell culture medium B to promote the cells to produce secretion factor;
F3. Centrifugate and filtrate the collated culture solution to obtain the secretion product.

Preferably, said mesenchymal stem cell culture medium B contains basic fibroblast growth factor and platelet derived growth factor.

The second objective of the present Invention Is to provide a method for extracting the secretion product of mesenchymal stem cells, said method comprises the following steps:
A'. Digest the mesenchymal stem cells with enzyme digestion solution;
B' Add culture medium into the collected single cells for inoculation and culturing, and add In mesenchymal stem cell culture medium B to promote the cells to produce secretion factor;
C' Centrifugate and filtrate the collected culture solution to obtain the secretion product.

Preferably, said step A' further comprises:
A'1, Digest the mesenchymal stem cells with 100ml digestion solution containing 0.05g tripsin and 0.02g EDTA•4Na;
A'2. When the mesenchymal stem cells turn round and detach, terminate the digestion with 1640 medium containing bovine serum.

Preferably, said mesenchymal stem cell culture medium B comprises:

| | |
|---|---|
| Basic fibroblast growth factor | 1 ng/ml -100 ng/ml medium |
| Platelet derived growth factor | 1 ng/ml - 100 ng/ml medium |

wherein, said medium Is prepared with components of the following weight percentage:

| | |
|---|---|
| DMEM medium | 96.9% |
| Sodium pyruvate | 1% |
| Antibiotic and glutamine | 1% |
| Non-essential amino acid | 1% |
| Antioxidant | 0.1 % |

Preferably, the step of adding in mesenchymal stem cell culture medium B to promote the cells to produce secretion factor in said step B' comprises: when the cell layer in the culture flask expands to reach the set volume, take out the culture solution and wash with phosphate buffer; then add in mesenchymal stem cell culture medium B and stimulate the mesenchymal stem cells to produce secretion factor.

Preferably, the step of adding In culture medium for inoculation and culturing in said step B' comprises:
adding mesenchymal stem cell culture medium into isolated single cells, shaking well and adjusting the cell density to 5-8x10⁴ cells/ml;
Inoculate the adjusted cell culture Into multiple culture flasks and Incubate in an incubator with 5-10% CO₂, 37°C, and saturated humidity.

The third objective of the present invention is to provide the secretion product produced according to the above method of the present invention for extracting the secretion product of mesenchymal stem cells.

The fourth objective of the present Invention Is to provide a use In human cosmetic aspect of the secretion product produced according to the above method of the present invention for extracting the secretion product of mesenchymal stem cells.

The fifth objective of the present invention is to provide a use in the aspect of healthcare product of the secretion product produced according to the above method of the present invention for extracting the secretion product of mesenchymal stem cells.

The sixth objective of the present invention is to provide an epidermal cell repairing lotion containing components of the following weight percentage:

| | |
|---|---|
| Solvent | 50-94% |
| Preservative | 0-0.5% |
| Humectants | 0-3% |
| Secretion product | 0.5-25% |
| Collagen | 5-25% |

Preferably, said preservative Includes: Imidazolidinyl urea, propylparaben and/or methylparaben; said humectants Include glycerol and/or hyaluronic acid.

It can be known from the above that in the process of the present invention for extracting mesenchymal stem cells from human or animal embryo, more single cells can be obtained by using mixed enzyme digestion solution as the digestion solution, while reducing the extent of cell damage to the minimum; In addition, for mesenchymal stem cells selected by adherence purification and immunomagnetic separation, the purity is increased. Furthermore, by adding mesenchymal stem cell culture medium A to the Isolated single cells and Inoculating and culturing the cells, cell growth and proliferation are facilitated. Moreover, the addition of mesenchymal stem cell culture medium B during the process of culturing mesenchymal stem cells can stimulate the mesenchymal stem cells to produce more secretion factors, thus, the secretion product extracted from the mesenchymal stem cells contains more cell secretion factors.

### Brief Description of Drawings

The present invention Is further Illustrated with drawings and examples as indicated below, in the drawings:
Figure 1 is the flowchart for the method of extracting human or animal mesenchymal stem cells in one example of the present invention;
Figure 2 is a picture representing the adherent growth of primary cells, after being digested with enzyme digestion solution in one example of the present invention;
Figure 3 is a picture representing the adherent growth of primary cells, after being digested with enzyme digestion solution in another example of the present Invention;
Figure 4 is a picture representing the adherent growth of primary cells, after being digested with enzyme digestion solution in a further example of the present invention;
Figure 5 is a picture representing the adherent growth state of the cells obtained after cell inoculation and culturing using mesenchymal stem cell culture medium A in one example of the present invention;
Figure 6 is a picture representing the adherent growth state of the cells obtained after cell inoculation and culturing using mesenchymal stem cell culture medium A in another example of the present invention;
Figure 7 is a picture representing the adherent growth state of the cells obtained after cell Inoculation and culturing using mesenchymal stem cell culture medium A in a further example of the present invention;
Figure 8 Is a picture representing the adherent growth state of the cells obtained after cell inoculation and culturing using the culture medium of the prior art;
Figure 9 is the flowchart for the method of extracting mesenchymal stem cells from human or animal embryos in another example of the present invention;
Figure 10 is a picture representing the characterization of the expression of the proteins on the surface of mesenchymal stem cells in one example of the present invention;
Figure 11 is a picture representing the differentiation of the placenta mesenchymal stem cells Into fat cells, chondrocytes and osteoblasts induced with cytochemical methods in one example of the present invention;
Figure 12 is the flowchart for the method of extracting the secretion product of mesenchymal stem cells In one example of the present Invention;
Figure 13 is a picture representing the identification of the secretion factors of mesenchymal stem cells In one example of the present invention;
Figure 14 shows that in one example of the present invention, after examination it was found that the secretion product of mesenchymal stem cells contains a large number of cell growth factor groups and collagen molecule groups;
Figure 15 is a picture representing the effect of the secretion product of mesenchymal stem cells in epidermis repairing in one example of the present invention;
Figure 16 Is a picture representing the repair of burn wound by epidermal cell repairing lotion in one example of the present invention.
Figure 17 is a picture representing the use of epidermal cell repairing lotion in human cosmetic aspect in one example of the present invention.

### Best Mode for Carrying out the invention

In order to make the objectives, technical solutions and advantages of the present invention clearer, drawings and examples are employed below to further illustrate the invention. It should be understood that the particular examples described here are only used to explain the invention and are not limiting.

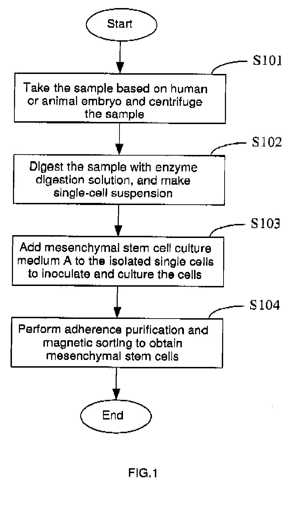

Figure 1 shows the flowchart for the method of extracting human or animal mesenchymal stem cells in one example of the present invention, comprising the following steps: in the step S101, human or animal embryo derived sample was taken and treated with centrifugation. In the step S102, the sample was digested with enzyme digestion solution to make single cell suspension. In the step S103, mesenchymal stem cell culture medium A was added into the isolated single cells to inoculate and culture the cells. In the step S104, the cells were treated with adherent purification and mesenchymal stem cells were obtained by magnetic sorting.

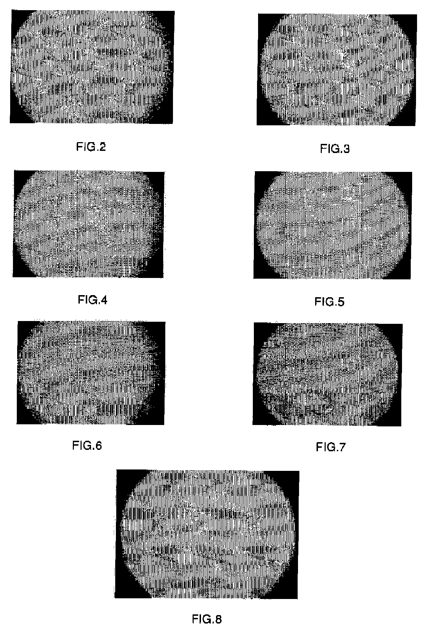

In a preferred example of the present invention, said step S102 further comprises:
1. The supernatant was aspirated off after centrifugation. 0.375g tripsin, 0.125g EDTA and 0.25g collagenase IV were dissolved in 1L normal saline to make the enzyme digestion solution of the invention. Then, the prepared enzyme digestion solution was added into a 30 ml centrifuge tube and the digestion was performed in a thermostatic waterbath shaker at 37°C for 1.5 - 2h, the adherent growth state of the obtained primary cells was as shown in figure 2. In anther example of the invention, 0.375g tripsin and 0.125g EDTA may be dissolved in 500 ml D-HBSS first, and then 0.25g collagenase IV was dissolved in 500 ml normal saline, after that, the 500 ml D-HBSS and the 500 ml normal saline was mixed to make 1L enzyme digestion solution of the invention. In other examples of the present Invention, those skilled in the art may also use other methods or buffers to prepare the enzyme digestion solution of the invention.
2. The digested solution was diluted with normal saline 3 -5 times of Its volume, well shaken and transferred with electric pipette through a 100µm cell strainer to make single cell suspension.

In another example of the invention, equal volumes of 100 ml D-HBSS containing 0.05g tripsin and 0.02g EDTA·4Na and 100 ml normal saline containing 0.01 g collagenase IV may be mixed to make the enzyme digestion solution of the invention. Then, 30 ml of the prepared enzyme digestion solution was added into a centrifuge tube and the digestion was performed In a thermostatic waterbath shaker at 37°C for 1.5 - 2h, the adherent growth state of the obtained primary cells was as shown in figure 3. In other examples of the invention, normal saline may be substituted with, for example, PBS. In the preferred example of the Invention, the tripsin-EDTA (0.05%) produced by the company GIBCO may be mixed with collagenase digestion solution (1mg collagenase IV/ml normal saline) at the volume ratio of 1:1 for preparation.

In a further example of the present invention, 0.125g tripsin, 0.075g EDTA and 0.75g collagenase IV may be dissolved in the mixture of 500 ml normal saline and 500 ml D-HBSS to prepare the enzyme digestion solution of the Invention. Then. 30 ml of the prepared enzyme digestion solution was added Into a centrifuge tube and the digestion was performed in a thermostatic waterbath shaker at 37°C for 1.5- 2h, the adherent growth state of the obtained primary cells was as shown In figure 4.

In other examples of the Invention, the enzyme digestion solution of the Invention may also be made according to other proportions, buffers or methods. For example, 0.2g tripsin and 0.05g EDTA·4Na were dissolved in 500 ml D-HBSS, and at the same time, 0.5g collagenase IV was dissolved in 500 ml normal saline or PBS, then the 500 ml D-HBSS was mixed with the 500 ml normal saline to make 1L enzyme digestion solution of the invention.

Repeated experiments showed that mixing equal volumes of the tripsin-EDTA (0.05%) produced by the company GIBCO and collagenase digestion solution (1 mg collagenase IV/ml normal saline) and digesting for 1.5-2h can reduce the digestion damage of the enzyme to the cells to its minimum, and can also obtain the maximal number of single cells. Thus, during the digestion process, a good control of the proportion of the two enzymes and the time of the digestion were essential for the successful Isolation of mesenchymal stem cells from human placenta.

In a preferred example of the present Invention, said mesenchymal stem cell culture medium A contained medium, basic fibroblast growth factor, epidermal growth factor, and platelet derived growth factor. In the medium, the concentration of the basic fibroblast growth factor was 1ng/ml, the concentration of the epidermal growth factor was 1 ng/ml, and the concentration of the platelet derived growth factor was 1 ng/ml. In this example, the medium contained 97% weight percentage of DMEM medium, 1% weight percentage of umbilical cord blood plasma, 1% weight percentage of penicillin-streptomycin- glutamine solution, and 1% weight percentage of non-essential amino acids. The mesenchymal stem cell culture medium A described above was used to inoculate and culture the cells, and the adherent growth state of the cells obtained was as shown in figure 5.

In the present invention, unless otherwise stated, various components in the medium of the above described mesenchymal stem cell culture medium A and mesenchymal stem cell culture medium B were bought from the U.S. company GIBCO.

In another preferred example of the invention, said mesenchymal stem cell culture medium A contained medium, basic fibroblast growth factor, epidermal growth factor, and platelet derived growth factor. In the medium, the concentration of the basic fibroblast growth factor was 25 ng/ml, the concentration of the epidermal growth factor was 25 ng/ml, and the concentration of the platelet derived growth factor was 25 ng/ml. In this example, the medium contained 77.9% weight percentage of DMEM medium, 20% weight percentage of umbilical cord blood plasma, 1% weight percentage of penicillin-streptomycin-glutamine solution, 1% weight percentage of non-essential amino acids, and 0.1% weight percentage of β- mercaptoethanol. The mesenchymal stem cell culture medium A described above was used to inoculate and culture the cells, and the adherent growth state of the cells obtained was as shown in figure 6.

In another preferred example of the invention, said mesenchymal stem cell culture medium A contained medium, basic fibroblast growth factor, epidermal growth factor, and platelet derived growth factor. In the medium, the concentration of the basic fibroblast growth factor was 100 ng/ml, the concentration of the epidermal growth factor was 100 ng/ml, and the concentration of the platelet derived growth factor was 100 ng/ml. In this example, the medium contained 67.9% weight percentage of DMEM medium, 20% weight percentage of umbilical cord blood plasma, 1% weight percentage of penicillin-streptomycin-glutamine solution, 1% weight percentage of non-essential amino acids, and 0.1% weight percentage of β- mercaptoethanol. The mesenchymal stem cell culture medium A described above was used to Inoculate and culture the cells, and the adherent growth state of the cells obtained was as shown in figure 7.

Figure 8 is a picture representing the adherent growth state of the cells obtained after cell inoculation and culturing using the culture medium of the prior art. From the comparison of the adherent growth state of the cells shown in figures 5-8, it can been seen that after culturing the cells with the mesenchymal stem cell culture medium A, cell growth was obviously facilitated.

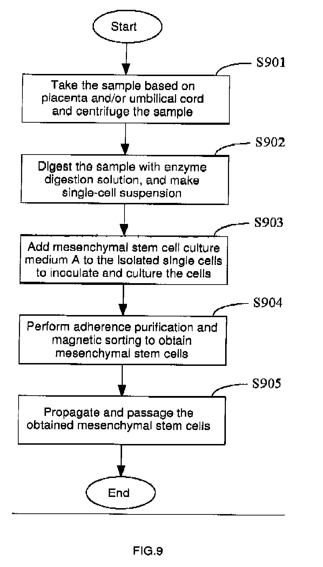

Figure 9 Is the flowchart for the method of extracting mesenchymal stem cells from human or animal embryos in one example of the present Invention. As shown in figure 9, in the step S901. animal placenta and/or umbilical cord derived sample was taken and treated with centrifugation. In one example of the invention, detailed procedure for carrying out said step is: 1, the membrane on the outside of a fresh placenta was removed with scissors, dark red tissues that were inside were taken. It was most preferred to take tissues with dense blood vessels, where the blood vessels interwove to form a root like structure. The blood in the tissue was rinsed off with normal saline (with heparin, 2 vials of heparin for 500 ml saline, 12500IU/vial, final concentration: 50 unit heparin/ml saline), the tissue was then rinsed once with heparin-free normal saline, after that, the tissue was cut into pieces with scissors (the smaller the pieces the better). The way to treat the umbilical cord was: the umbilical cord was cut to get fragments of about 2cm, the blood was rinsed off with saline containing heparin, then the umbilical cord fragments were cut open longitudinally and the blood inside was rinsed off, the umbilical cord was rinsed once again with heparin-free normal saline and cut into pieces with scissors. Of course, the invention Is not limited to the above procedure, other parts of an embryo may also be chosen as the material of the invention. 2. The sample was transferred into a centrifuge tube containing normal saline and treated with centrifugation. In a preferred example, the collected sample was placed into a 50 ml centrifuge tube containing normal saline and centrifuged at 1200rpm for 10 min.

In the step S902, the sample was digested with the enzyme digestion solution to make single cell suspension. In one example of the invention, detailed procedure for carrying out said step is: 1. after centrifugation, the supernatant was aspirated off, tripsln-EDTA (0.05%) and collagenase IV (1mg/ml) was mixed at the volume ratio of 1:1, 30 ml of which was added into a centrifuge tube and the digestion was performed In a thermostatic waterbath shaker at 37°C for 1.5 - 2h. Certainly, other preparation schemes may also be used in the step of digestion, see above for detailed information; 2. the digested solution was diluted with normal saline 3 -5 times of its volume, well shaken and transferred with electric pipette through a 100µm cell strainer to make single cell suspension.

In the step S903, isolated single cells were obtained from the single cell suspension, and mesenchymal stem cell culture medium A was added into the isolated single cells to inoculate and culture the cells. In one example of the invention, detailed procedure for carrying out said step is: 1. single cell suspension was centrifuged at 1600 rpm for 10 min and the supernatant was aspirated off; 2. the mesenchymal stem cell culture medium A was added, well-shaken and adjusted till the cell density reached to 5-8x10⁴ cells/ml; 3. the adjusted solution was Inoculated into multiple-well plates (such as 6-well plates) and incubated In a incubator with 5-10% CO₂, 37°C, and saturated humidity, Of course, other parameters may also be used in the invention and the above embodiments are not used to limit the scope of protection of the present invention. Said mesenchymal stem cell culture medium A therein may be chosen from any of the examples described above.

In the step S904, the cells were treated with adherent purification and mesenchymal stem cells were obtained by magnetic sorting. In one example of the invention, detailed procedure for carrying out said step is:

### 1, Adherent purification treatment, specifically comprising:

Non-adherent cells were taken out with a pipette, and normal saline was added in to wash, then it was changed into new mesenchymal stem cell culture medium A to continue the culturing. Digestion was performed with tripsin-EDTA (0.05%) when the adherent cells expanded to reach 70% of the volume of the culture flask and single cells were obtained.

In the present invention, single cells were obtained by ordinary centrifugation (1600r/min) of the single cell suspension obtained after the digestion with tripsin-EDTA (0.05%). In the population of such single cells, there were many cells with different biological properties, some dead cells were also mixed therein. Then, selection was carried out with the culture medium used to culture mesenchymal stem cells, those cells that survived and were able to grow adhering to the wall were mostly mesenchymal stem cells. A large number of cells that were not mesenchymal stem cells can be removed by adherent purification.

### 2. Magnetic sorting, specifically comprising:

immunomagnetic separation method was used to select CD271 and CD73 double-positive cell population from said single cells, and these were mesenchymal stem cells isolated from the placenta. A large number of mesenchymal stem cells that were alive and of high purity can be obtained within the shortest time by magnetic sorting.

In the step S905, the obtained mesenchymal stem cells were propagated and passaged, detailed procedure for carrying out said step Is: 1. the medium in the flask was removed with a pipette. 2. a small amount of phosphate buffer was added to gently wash once and was aspirated off, so that the pH value was equilibrated. 3. tripsin-EDTA (0.05%) was added, so much as the enzyme solution was enough to cover the bottom of the flask, it was observed under the microscope, and the digestion can be terminated with 1640 medium containing fetal bovine serum when most of the cells turned round and detached. 4. the culture medium In the flask was drawn with a pipette and the cell layer was pipetted repeatedly, the cell suspension was collected in a 50 ml centrifuge tube and the flask was washed once again with PBS. 5. After the cell suspension was centrifuged at 1200rpm for 8min, the supernatant was removed, and mesenchymal stem cell culture medium A was added. The cells were mixed well and passed at the ratio of 1:n to propagate in the flask, so that a large number of placenta mesenchymal stem cells were obtained, and wherein n>1, for example a typical value for n is 4.

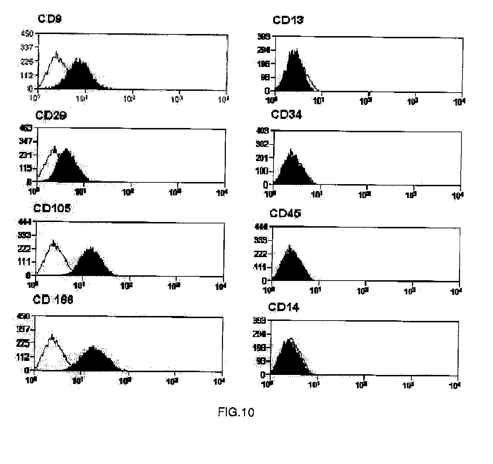

Figure 10 is a picture representing the characterization of the expression of the proteins on the surface of mesenchymal stem cells in one example of the present invention. FlOW Cytometer was used in the present invention to examine the placenta mesenchymal stem cells and demonstrate the expression of cytokine. It can be seen that the isolated and cultured placenta mesenchymal stem cells expressed surface proteins CD9, CD29, CD105, and CD186 and did not express surface proteins CD34, CD13, CD14, and CD45, this population of cells has stable biological property and the cell homogenicity reached 95% and 98% after the cells proliferated for one generation and two generations, respectively. In this figure, the hollow light-colored curve represents negative control for the expression of a cytokine, and the solid black pattern represents the positive ratio of the corresponding cytokine expressed in the cell population. The further away the solid black pattern is from the hollow light-colored curve, the higher the positive ratio of said cytokine.

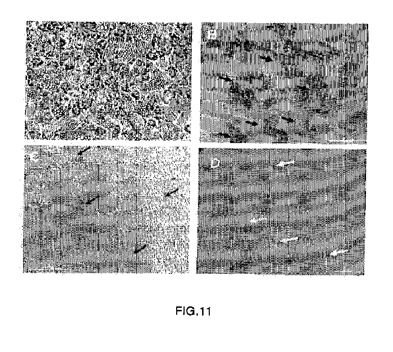

Figure 11 is a picture representing the differentiation of the placenta mesenchymal stem cells into fat cells, chondrocytes and osteoblasts induced with cytochemical methods in one example of the present invention. After continuous passage and culturing, and cryopreservation, placenta mesenchymal stem cells (PDMSC) are still multipotent to differentiate and they maintain normal karyotype nuclear telomerase activity, but they don't differentiate spontaneously easily. Under specific In vitro induction conditions, PDMSC can differentiate into various types of cells such as bone, cartilage, fat, tendon, muscle, and nerve. In figure 11, panel A shows that the addition of adipogenic inducer resulted In the differentiation of placenta mesenchymal stem cells Into adipocytes and the Oil Red staining result was positive, orange red lipid droplet could be seen In the cytoplasm. Panel B shows that after the addition of osteogenic inducer, placenta mesenchymal stem cells differentiated into osteoblasts, Von Kossa was performed to identify the osteoblasts. Dark brown cells could be seen from the Von Kossa staining and the intercellular matrix was full of black particles, suggesting the existence of mineralized matrix deposition. Panel C shows the addition of cartilage-inducing agent to induce the cells, and the expression of proteoglycan was shown by Alcian blue staining. Panel D shows that after the Induction of differentiation, the expression of cartilage-cell-specific collagen II was detected by immunochemical method-cartilage cells.

It is necessary to further illustrate the application of placenta mesenchymal stem cells as trophoblasts for human embryonic stem cells (hESC) and murine embryonic stem cells (mESC). As cytotrophoblasts, placenta mesenchymal stem cells (PDMSC) provide growth support for hESCs and mESCs and maintain the embryonic stem cells in their undifferentiated state. Currently, mice embryo fibroblasts (MEF) are used as the trophoblast for hESCs and mESCs. However, there are many limitations using the MEF as the trophoblast for stem cell growth: 1. the risk of contamination of animal origin exists; 2 many mice need to be sacrificed to extract a large number of MEFs. There are unique advantages for using PDMSCs derived from healthy human as trophoblast for embryonic stem cells: 1. since the material is taken from discarded placenta tissues of healthy human, sacrificing many mice is avoided; 2. the risk of contamination of animal origin is avoided; 2. it is more advantageous to use human PDMSCs as trophoblast of human embryonic stem cells for culturing human embryonic stem cells. Thus, as the origin of hESC trophoblast, PDMSC is a better choice than MEF.

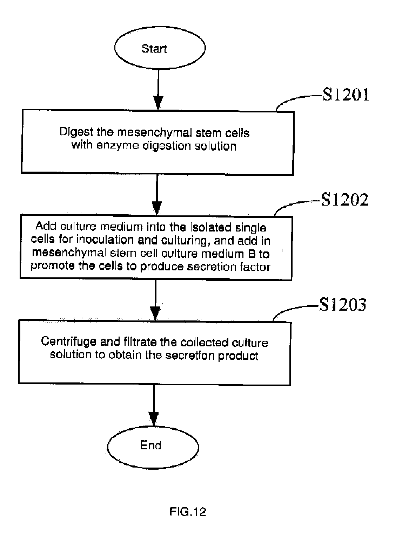

Figure 12 is the flowchart for the method of extracting the secretion product of mesenchymal stem cells In one example of the present invention. In the step S1201, the mesenchymal stem cells were digested with enzyme digestion solution, detailed procedure thereof comprises: 1. after mass amplification of the cells, trypsin-EDTA(0.05%) was added for digestion when the cells grew to about 80% of the 175cm² culture flask. 2. when said mesenchymal stem cells turned round and detached, the digestion was terminated with 1640 medium containing bovine serum.

In the step S1202, culture medium was added into the collected cells for Inoculation and culturing, and mesenchymal stem cell culture medium B was added to promote the cells to produce more secretion factors, the detailed procedure thereof comprises:
1. Culture medium was added into the collected cells for Inoculation and culturing. In a preferred example, detailed procedure of the above step is: mesenchymal stem cell culture medium was added into the collected cells, well shaken and adjusted till the cell density reached 5-8 x 10⁴ cells/ml; the adjusted solution was Inoculated Into the culture flask and cultured in an incubator with 5%CO₂, 37°C, and saturated humidity. In other examples of the present invention, the 5%CO₂ concentration may also be adjusted according to need, such as 10%, 8%, 6% etc.
2. Mesenchymal stem cell culture medium B was added to promote the cells to produce more secretion factors. In a preferred example, detailed procedural of the above step is: when the cell layer In the culture flask expanded to 80%, the culture medium was taken out and the cells were washed with phosphate buffer.

In the first preferred example of the present invention, said mesenchymal stem cell culture medium B comprises the medium, basic fibroblast growth factor, and platelet derived growth factor. In the medium, the concentration of basic fibroblast growth factor was 1 ng/ml, the concentration of platelet derived growth factor was 1 ng/ml. In this example, the medium contains 96.8% weight percentage of DMEM medium, 1 % weight percentage of penicillin-streptomycin-glutamine solution, 1% weight percentage of non-essential amino acids, 0.1% weight percentage of β- mercaptoethanol and 1 % weight percentage of sodium pyruvate.

In the second preferred example of the present invention, said mesenchymal stem cell culture medium B comprises the medium, basic fibroblast growth factor, and platelet derived growth factor. In the medium, the concentration of basic fibroblast growth factor was 25ng/ml, the concentration of platelet derived growth factor was 25ng/ml. In this example, the medium contains 96.8% weight percentage of DMEM medium, 1% weight percentage of penicillin-streptomycln-glutamine solution, 1% weight percentage of non-essential amino acids, 0.1% weight percentage of β- mercaptoethanol and 1% weight percentage of sodium pyruvate.

In the third preferred example of the present invention, said mesenchymal stem cell culture medium B comprises the medium, basic fibroblast growth factor, and platelet derived growth factor. In the medium, the concentration of basic fibroblast growth factor was 100ng/ml, the concentration of platelet derived growth factor was 100ng/ml. In this example, the medium contains 96.8% weight percentage of DMEM medium, 1% weight percentage of penicillin-streptomycin-glutamine solution, 1% weight percentage of non-essential amino acids, 0,1% weight percentage of β-mercaptoethanol and 1% weight percentage of sodium pyruvate.

In the step S1203, the collected culture solution was centrifuged and filtrated, and the secretion product was obtained. The detailed procedure of the above step is: 1, the culture solution was collected, and the supernatant was collected after centrifuging at 2000rpm for 8min. In a preferred example, the detailed procedure of the above step is: the culture solution was collected after culturing for 3 days. After centrifuging the harvested solution at 2000G for 8min, the cell debris was removed and the supernatant was collected. 2. the solution was filtrated with a filter of 0.22µm and the secretion product was obtained. In a preferred example, detailed procedure of the above step is : filtration was performed with a filter of 0.22µm, the filtered fluid was collected and stored under the condition of - 20°C for 2 days, and then placed under the condition of -80°C for long term storage and future use.

In the preferred example of the present invention, said method of extracting the secretion product of mesenchymal stem cells further comprises concentrating said secretion product. In an example, the detailed implementation of the concentration step comprises, first of all, preparing the ultrafiltration membrane: the ultrafiltration membrane was first rinsed with 75% ethanol and soaked for 10min; the edge of the membrane was held with forceps and the membrane was rinsed for three times with water for injection and then soaked overnight in water for injection in a clean container.
1. The harvested solution was added into an ultrafiltration cup till the working volume was reached, then the assembly was completed. In a preferred example, detailed procedure of the above step is: the membrane, the cup body and the mixing system were assembled according to the instructions of the ultrafiltration cup, the harvested solution was added till working volume was reached, then the cup cover and the protective case were fit on 2. nitrogen was Introduced and the filtration started, and the concentrated solution obtained after the filtration was collected. In a preferred example, detailed procedure of the above step is: nitrogen was introduced and the filtration started, It has to be noted that the pressure should be increased slowly In the beginning of the filtration and the magnetic stirrer was turned on once there was pressure; during the filtration, the nitrogen pressure cannot be over 55PSI; the pressure should be reduced slowly after the filtration was finished. Finally, the concentration was terminated when the harvested solution In the ultrafiltration cup was concentrated to 25 times, the concentrated solution was collected in a super-clean bench and stored In a -80°C refrigerator for future use.

When the mesenchymal stem cell culture medium B was not used, the protein concentration In the concentrated secretion products was 0.2mg/ml. When the mesenchymal stem cell culture medium B according to the above first example was used, the protein concentration in the concentrated secretion products was 0.3mg/ml; when the mesenchymal stem cell culture medium B according to the above second example was used, the protein concentration in the concentrated secretion products was 1.5mg/ml; when the mesenchymal stem cell culture medium B according to the above third example was used, the protein concentration in the concentrated secretion products was 2mg/ml.

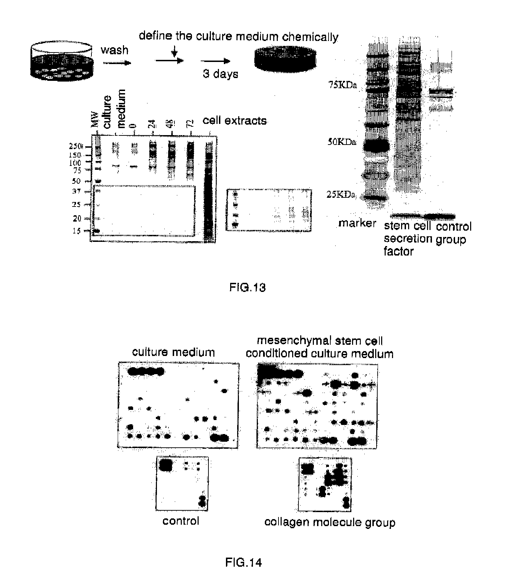

Figure 13 is a picture representing the identification of the secretion factors of mesenchymal stem cells In one example of the present invention. A large number of protein molecules were contained in placenta mesenchymal stem cell secretion factor products. During the culturing and proliferation process of placenta mesenchymal stem cells, the placenta mesenchymal stem cells will secrete many substances that promote cell growth and tissue regeneration, such as growth factors, proteins, active polypeptides. In this figure, the cytokines secreted by placenta mesenchymal stem cells revealed by silver staining were significantly different from the control group, there were a large number of specific protein molecules contained in the secretion product of placenta mesenchymal stem cells.

Figure 14 shows that in one example of the present invention, after examination, it was found that the secretion product of mesenchymal stem cells contained a large number of cell growth factor groups and collagen molecule groups. The proteomic of the secretion factors of placenta mesenchymal stem cells was assessed using cytokine antibody array. It was found that a large number of placenta mesenchymal stem cells secreted specific gene products, among these gene products, there were a lot of growth factor groups relating to cell growth and metabolism, for example: BDNF. EGF, FGF, FGF17, FGF4, FGF6, FGF7, FGF9, GDNF, HGF, IGFBp, PDGFB, PIGF, TGFB1, TGFb2, TNFRSF11 B, VEGF, IGF1, and LEP; and a lot of collagen molecule groups, for example: COL11A1, COL12A1, COL16A1, COL1A, COL3A1, COL4A1, COL5A and COL6A. These protein molecule groups are very Important for promoting cell growth and metabolism. In this Figure, after examination, It was found that the secretion product of placenta mesenchymal stem cells contained a large number of cell growth factor groups and collagen molecule groups, as Indicated by the arrows In the figure.

Here, It Is necessary to illustrate the functions of the secretion product of mesenchymal stem cells, their functions in the following aspects are mainly elucidated: epidermis repair, human cosmetic, regenerative medicine, healthcare products.

The present invention also provides an epidermal cell repairing lotion, comprising components of the following weight percentage: water 71 %, preservative 0.5%, humectants 3%, secretion product 5.5%, collagen 20%, wherein the preservative may include imidazolidinyl urea 0.15%, propylparaben 0.2% and methylparaben 0.15%, the humectants comprise glycerol 2% and hyaluronic acid 1% and the secretion product can be obtained by the methods described above.

In another example of the present invention, said epidermal cell repairing lotion comprises components of the following weight percentage: water 94%, humectants 0.5%, secretion product 0.5%, collagen 5%, wherein the humectants comprise glycerol 0.25% and hyaluronic acid 0.25%.

In another example of the present Invention, said epidermal cell repairing lotion comprises components of the following weight percentage: water 88%, imidazolidinyl urea 0.1%, propylparaben 0.2%, methylparaben 0.1%, glycerol 1%, hyaluronic acid 1%, secretion product 2%, collagen 7.5%.

In another example of the present invention, said epidermal cell repairing lotion comprises components of the following weight percentage: water 50%, secretion product 25%, collagen 28%.

In yet another example of the present invention, said epidermal cell repairing lotion comprises components of the following weight percentage: water 68.5%, Imidazolidinyl urea 0.3%, propylparaben 0.2%, glycerol 1%, hyaluronic acid 1%, secretion product 15%, collagen 14%,

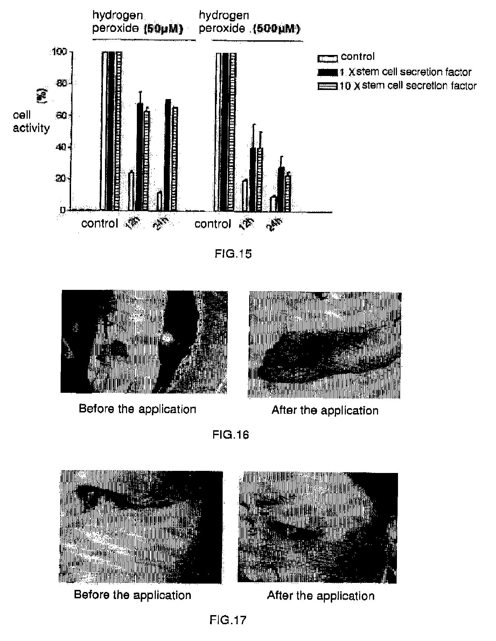

Because the secretion product of mesenchymal stem cells according to the present Invention has significant protective effects for skin fibroblasts, it has been shown in the experiments of resisting the damage of hydrogen peroxide and repairing fibroblasts that cell mortality caused by the damage of hydrogen peroxide can be significantly reduced after the addition of culture medium into placenta mesenchymal stem cell products (CM), and In turn the survival rate of the survived fibroblasts was increased. In the experiments of resisting the damage of hydrogen peroxide and repairing fibroblasts, cell mortality caused by the damage of hydrogen peroxide can be significantly reduced after the addition of culture medium into plaoenta Mesenchymal stem cell secretion products, and along with the Increase of the CM amount, the survival rate of its fibroblasts was also clearly increased (see figure 15).

By using the epidermal cell repairing lotion of the present invention, the division and proliferation of fibroblasts and vascular endothelial cells can be promoted and the synthesis and secretion of extracellular matrix are promoted, so that the growth of granulation tissue is promoted and the sinus tract becomes shallower; the division and proliferation of epidermal cells are facilitated, so that the epithelization of wound is promoted. When applying it to burn wound, it can promote division and proliferation of fibroblasts and vascular endothelial cells, as well as the synthesis and secretion of extracellular matrix, so that the growth of granulation tissue is promoted and the wound is shallower; it facilitates the division and proliferation of epidermal cells around the remaining hair follicle, sweat gland and wound, so as to facilitate the epithelization of wound, see figure 16.

The use of the secretion product of this invention in the aspect of human cosmetic is described below:

The cosmetic effect of the placental mesenchymal stem cell secretion product is achieved by utilizing the ability of the factors secreted by placental mesenchymal stem cells to increase the production of collagen and to stimulate the growth of skin fibroblast cells. That the skin is elastic is mainly because the collagen secreted by fibroblast cells in the dermis of the skin forms the scaffoid of the skin. This secretion product increases the volume of the collagen produced by skin fibroblast cells for several folds, at the same time the volume of fibroblast cells is also increased for more than 30 percent. Therefore, continuous production of autologous Collagen will cause the thickness and density of the skin dermis to increase, fill the wrinkles, eliminate scars and restore the elasticity and luster of the skin.

Usually, the secretion products of mesenchymal stem cells are added into a cosmetic formula. The various active ingredients, especially the cell growth factor group and the collagen group, contained in these secretion products can promote hydroxyproline synthesis, facilitate the synthesis of collagen and collagenase, secrete collagen substances, hyaluronic acid and glycoproteins, adjust collagenous fibers, so that it has the effects of moisturizing the skin, enhancing skin elasticity, reducing skin wrinkles and preventing skin aging. The aforementioned epidermal cell repairing lotion of the invention can also be used to reduce skin wrinkles and prevent skin aging, its effects are shown In figure 17. It clearly improved skin elasticity and the skin became tight; wrinkles at the canthus and forehead became shallower, the skin was smooth and soft, and pigmentation was improved.

The use of the secretion product in the aspect of healthcare products is described below:

Currently, the most ideal high-technology nutri-cosmetics used to repair damaged skin and red-blood-streak exposing skin usually are rich in various kinds of active factors, such as basic fibroblast growth factor (FGF) and epidermal growth factor (EGF). Placenta mesenchymal stem cells can secrete large amount of these growth factors. These growth factors not only can adjust the differentiation and proliferation of epidermal cells and promote skin renewal, but also can increase the elasticity of the skin, restore the moisture content of the skin. It contains liquorice and other calming and repairing ingredients and repairs the stratum corneum. In addition, the abundant grape seed OPC is an effective antl-oxidant and anti-free-radical component, which can repair cells, intensify the repairing of the stratum corneum, and can also protect the stratum corneum and prevent the oxidation and yellowing of the stratum corneum. Therefore, supplementing ordinary cosmetic products with the mesenchymal stem cell secretion product of the invention and grape seed OPC will surely multiply the efficacy. According to the teachings of this invention, people skilled in the art are aware of various methods for applying the secretion product of this invention to current cosmetic products, healthcare products and medicaments, and can obtain various desired cosmetic products, healthcare products and medicaments by these methods, so that it is not further described here.

Those described above are just preferred examples of the present invention, but are not used to limit the invention. Any modification, alternative, improvement and the like made within the spirit and principle of this invention should all be included in scope of protection of this invention.

## Claims

1. A method for extracting mesenchymal stem cell from human or animal embryo, **characterized in that**, said method comprises the following steps:
A. a sample is taken based on human or animal embryo, and centrifuge the sample;
B. digest the sample with enzyme digestion solution and make single cell suspension, wherein said enzyme digestion solution comprises trypsin-EDTA and collagenase IV;
C. add mesenchymal stem cell culture medium A to the isolated single cells, inoculate and culture the cells;
D. treat the cells with adherence purification and obtain mesenchymal stem cells by magnetic sorting.

2. Method according to claim 1 for extracting mesenchymal stem cell from human or animal embryo, **characterized in that**, the enzyme digestion solution In said step B comprises:
| | |
|---|---|
| Trypsin | 0.125g/L∼0.375g/L; |
| EDTA | 0.075g/L~0.125g/L; and |
| Collagenase IV | 0.25g/L∼0.75g/L. |

3. Method according to claim 2 for extracting mesenchymal stem cell from human or animal embryo, **characterized in that**, the preparation of the enzyme digestion solution in said step B comprises:
mixing 100ml D-HBSS containing 0.05g trypsin and 0.02g EDTA•4Na with 100ml normal saline containing 0.01g collagenase IV at the volume ratio of 1;1.

4. Method according to claim 1 for extracting mesenchymal stem cell from human or animal embryo, **characterized in that**, said mesenchymal stem cell culture medium A comprises:
| | |
|---|---|
| Basic fibroblast growth factor | 1 ng/ml∼100ng/ml medium |
| Epidermal growth factor | 1 ng/ml∼100ng/ml medium |
| Platelet derived growth factor | 1 ng/ml∼100ng/ml medium |
wherein, said medium comprise components of the following weight percentage:
| | |
|---|---|
| DMEM medium | 68%∼97% |
| Umbilical cord blood plasma | 1%∼30% |
| Antibiotic and glutamine | 1% |
| Non-essential amino acid | 1% |
| Anti-oxidant | 0-0.1% |

5. Method according to claim 4 for extracting mesenchymal stem cell from human or animal embryo, **characterized in that**, the adherence purification treatment In said step D comprises:
using a pipette to take out non-attached cells, adding normal saline to wash and exchanging to new mesenchymal stem cell culture medium A to continue culturing, digesting the cells with trysin-EDTA to obtain single cell suspension when the attached cells propagate to reach the defined volume of the culture flask.

6. Method according to claim 5 for extracting mesenchymal stem cell from human or animal embryo, **characterized in that**, the magnetic sorting in said step D comprises:
using immunomagnetic separation to select CD271 and CD73 double-positive cell population from said single cells, namely mesenchymal stem cells Isolated from the embryo.

7. Method according to any one of claims 1-6 for extracting mesenchymal stem cell from human or animal embryo, **characterized in that**, after said step D it further comprises:
E. propagating and passaging the obtained mesenchymal stem cells; and/or
F. extracting the secretion product of the mesenchymal stem cells.

8. Method according to claim 7 for extracting mesenchymal stem cell from human or animal embryo, **characterized in that**, said step F comprises:
F1. digesting the mesenchymal stem cells with enzyme digestion solution;
F2. adding culture medium into the collected cells for inoculation and culturing, and adding mesenchymal stem cell culture medium B to stimulate the cells to produce secretion factors;
F3. centrifuging and filtrating the collected culture solution, and obtaining the secretion product.

9. Method according to claim 8 for extracting mesenchymal stem cell from human or animal embryo, **characterized in that**, said mesenchymal stem cell culture medium B comprises basic fibroblast growth factor and platelet derived growth factor.

10. Method for extracting the secretion product of mesenchymal stem cells, **characterized in that**, said method comprises the following steps:
A' digesting the mesenchymal stem cells with enzyme digestion solution;
B' adding culture medium into the collected single cells for inoculation and culturing, and adding mesenchymal stem cell culture medium B to stimulate the cells to produce secretion factors;
C' centrifuging and filtrating the collected culture solution, and obtaining the secretion product.

11. Method according to claim 10 for extracting the secretion product of mesenchymal stem cells, **characterized In that**, said step A' further comprises:
A'1. digesting the mesenchymal stem cells using 100mL digestion solution containing 0.05g trypsin and 0.02g EDTA•4Na;
A'2. terminating the digestion with 1640 medium containing bovine serum, when the mesenchymal stem cells turn round and detach.

12. Method according to claim 10 for extracting the secretion product of mesenchymal stem cells, **characterized in that**, said mesenchymal stem cell culture medium B comprises:
| | |
|---|---|
| Basic fibroblast growth factor | 1 ng/ml∼100 ng/ml medium |
| Platelet derived growth factor | 1 ng/ml∼100 ng/ml medium |
wherein, said medium Is prepared with components of the following weight percentage:
| | |
|---|---|
| DMEM medium | 96.9% |
| Sodium pyruvate | 1% |
| Antibiotic and glutamine | 1% |
| Non-essential amino acid | 1% |
| Anti-oxidant | 0.1% |

13. Method according to claim 12 for extracting the secretion product of Mesenchymal stem cells, **characterized in that**, in said step B', the step of adding mesenchymal stem cell culture medium B to stimulate the cells to produce secretion factors comprises: when the cell layer in the culture flask expands to reach the defined volume, take out the culture medium and wash with phosphate buffer; then add mesenchymal stem cell culture medium B and stimulate the mesenchymal stem cells to produce secretion factors.

14. Method according to claim 10 for extracting the secretion product of mesenchymal stem cells, **characterized in that**, in said step B', the step of adding In culture medium for inoculation and culturing comprises: adding mesenchymal stem cell culture medium to the isolated single cells, shaking well and adjusting cell density to 5∼8x10⁴ cells/ml;
inoculating the adjusted cell solution into several culture flasks, culturing in incubators with 5∼10% CO₂, 37°C and saturated humidity.

15. Secretion product produced by the method according to any one of claims 10-14.

16. Use of the secretion product produced according to claim 15 in the aspect of human cosmetic.

17. Use of the secretion product produced according to claim 15 in the aspect of healthcare products.

18. An epidermal cell repairing lotion, **characterized In that**, it comprises ingredients of the following weight percentage:
| | |
|---|---|
| solvent | 50-94% |
| preservatives | 0-0.5% |
| humectants | 0-3% |
| secretion product | 0.5-25% |
| collagen | 5-25% |
